(19) Europäisches Patentamt
European Patent Office
Office européen des brevets

(11) Veröffentlichungsnummer: **0 048 828**
**A1**

(12) **EUROPÄISCHE PATENTANMELDUNG**

(21) Anmeldenummer: **81106723.0**

(51) Int. Cl.³: **C 07 D 233/60**, A 61 K 31/415

(22) Anmeldetag: **28.08.81**

(30) Priorität: **09.09.80 DE 3033917**

(43) Veröffentlichungstag der Anmeldung: **07.04.82**
**Patentblatt 82/14**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Reiser, Wolf, Dr., Klebitzweg 17, D-5600 Wuppertal 1 (DE)**
Erfinder: **Elbe, Ludwig, Dr., Dasnöckel 59, D-5600 Wuppertal 11 (DE)**
Erfinder: **Büchel, Karl Heinz, Prof. Dr., Dabringhausenerstrasse 42, D-5093 Burscheid (DE)**
Erfinder: **Plempel, Manfred, Dr., Palkestrasse 5, D-5600 Wuppertal 1 (DE)**

(54) **Substituierte 2,4-Dichlorphenyl-imidazolyl-vinyl-carbinole, Verfahren zu ihrer Herstellung sowie antimikrobielle Mittel, die diese Stoffe enthalten.**

(57) Substituierte 2,4-Dichlorphenyl-imidazolyl-vinyl-carbinole der allgemeinen Formel I

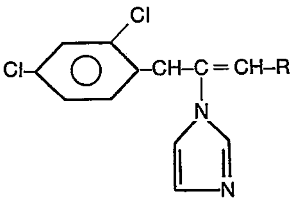

(I)

in welcher

R für Alkyl, gegebenenfalls substituiertes Cycloalkyl und Cycloalkenyl, gegebenenfalls substituiertes Cycloalkylalkyl und Cycloalkenylalkyl, gegebenenfalls substituiertes Alkenyl und Alkinyl, gegebenenfalls substituiertes Phenyl, sowie gegebenenfalls im Alkylteil und im Phenylteil substituiertes Phenylalkyl steht,

und deren physiologisch verträglichen Säureadditionssalze haben antimykotische Wirkung und sollen als Arzneimittel verwendet werden. Sie werden nach an sich bekannten Methoden hergestellt.



ACTORUM AG

BAYER AKTIENGESELLSCHAFT 5090 Leverkusen, Bayerwerk
Zentralbereich Si/W
Patente, Marken und Lizenzen Ia

Substituierte 2,4-Dichlorphenyl-imidazolyl-vinyl-carbinole, Verfahren zu ihrer Herstellung sowie antimikrobielle Mittel, die diese Stoffe enthalten.

Die vorliegende Erfindung betrifft neue substituierte 2,4-Dichlorphenyl-imidazolyl-vinyl-carbinole, ein Verfahren zu ihrer Herstellung sowie ihre Verwendung als antimikrobielle Mittel, insbesondere als Antimykotika.

Es ist bereits bekannt geworden, daß bestimmte 1-Phenyl-2-imidazolyl-4,4-dimethyl-1-penten-3-ole, wie z.B. das 1-(4-Chlorphenyl)-2-(imidazol-1-yl)-4,4-dimethyl-1-penten-3-ol, gute fungizide Eigenschaften besitzen (vergleiche DE-OS 28 38 847). Die Wirkung dieser Verbindungen gegen humanpathogene Pilze ist jedoch nicht immer voll befriedigend.

Es wurden neue substituierte 2,4-Dichlorphenyl-imidazolyl-vinyl-carbinole der allgemeinen Formel

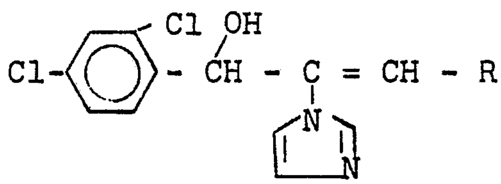

(I)

in welcher

R für Alkyl, gegebenenfalls substituiertes Cycloalkyl und Cycloalkenyl, gegebenenfalls substituiertes Cycloalkylalkyl und Cycloalkenylalkyl, gegebenenfalls substituiertes Alkenyl und Alkinyl, gegebenenfalls substituiertes Phenyl, sowie gegebenenfalls im Alkylteil und im Phenylteil substituiertes Phenylalkyl steht,

und deren physiologisch verträglichen Säureadditions-Salze gefunden.

Die Verbindungen der Formel (I) können in zwei geometrischen Isomerenformen (E- und Z-Isomeres) vorliegen, je nach Anordnung der Gruppen, die an die Doppelbindung gebunden sind; vorzugsweise fallen sie in einem wechselnden E,Z-Isomerenverhältnis an. Außerdem ist ein asymmetrisches Kohlenstoffatom vorhanden, so daß die Verbindungen der Formel (I) zusätzlich in zwei optischen Isomerenformen anfallen; vorzugsweise fallen sie als Racemate an. Die vorliegende Erfindung betrifft sowohl die einzelnen Isomeren als auch die Isomeren-Gemische.

Weiterhin wurde gefunden, daß man die substituierten 2,4-Dichlorphenyl-imidazolyl-vinyl-carbinole der Formel (I) erhält, wenn man 2,4-Dichlorphenyl-imidazolyl-vinyl-ketone der Formel

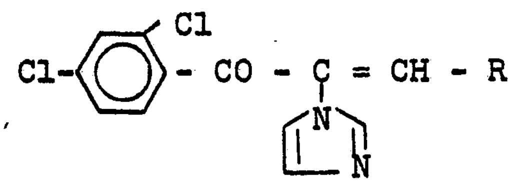

(II)

in welcher

R die oben angegebene Bedeutung hat,

nach bekannten Methoden in üblicher Weise reduziert.

An die so erhaltenen Verbindungen der Formel (I) kann gegebenenfalls anschließend eine Säure addiert werden.

Die Verbindungen der Formel (I) können weiterhin an der CH(OH)-Gruppe in üblicher und bekannter Weise derivatisiert werden, z.B. zu Ether-, Ester- und Carbamoyl-Derivaten.

Die neuen substituierten 2,4-Dichlorphenyl-imidazolyl-vinyl-carbinole der Formel (I) weisen starke antimykotische Eigenschaften auf. Dabei zeigen überraschenderweise die erfindungsgemäßen Verbindungen ein allgemein besseres Wirkungsspektrum als die aus dem Stand der Technik bekannten 1-Phenyl-2-imidazolyl-4,4-dimethyl-

-1-penten-3-ole, wie z.B. das 1-(4-Chlorphenyl)-2-(imidazol-1-yl)-4,4-dimethyl-1-penten-3-ol, welches chemisch naheliegende Verbindungen sind. Die erfindungsgemäßen Stoffe stellen somit eine Bereicherung der Pharmazie dar.

Die erfindungsgemäßen substituierten 2,4-Dichlorphenyl-imidazolyl-vinyl-carbinole sind durch die Formel (I) allgemein definiert. In dieser Formel steht R vorzugsweise für geradkettiges oder verzweigtes Alkyl mit 1 bis 29, insbesondere bis 18, Kohlenstoffatomen; für gegebenenfalls durch Alkyl mit 1 bis 4 Kohlenstoffatomen substituiertes Cycloalkyl und Cycloalkenyl mit jeweils 5 bis 7 Kohlenstoffatomen; für gegebenenfalls durch Alkyl mit 1 bis 4 Kohlenstoffatomen substituiertes Cycloalkylalkyl und Cycloalkenylalkyl mit jeweils 5 bis 7 Kohlenstoffatomen im Cycloalkyl- bzw. Cycloalkenyl-Teil und jeweils 1 bis 6 Kohlenstoffatomen im geradkettigen oder verzweigten Alkyl-Teil; für gegebenenfalls substituiertes geradkettiges oder verzweigtes Alkenyl und Alkinyl mit jeweils bis zu 6 Kohlenstoffatomen, wobei als Substituenten genannt seien: Hydroxy, Alkoxy mit 1 bis 4 Kohlenstoffatomen, sowie Phenyl, das gegebenenfalls substituiert sein kann durch Halogen und Alkyl mit 1 bis 4 Kohlenstoffatomen; für gegebenenfalls substituiertes Phenyl, wobei als Substituenten infrage kommen: Halogen, Alkyl mit 1 bis 4 Kohlenstoffatomen, Halogenalkyl mit 1 bis 2 Kohlenstoffatomen und 1 bis 5 gleichen oder verschiedenen Halogenatomen, wie insbesondere Fluor- und Chloratomen, sowie gegebenenfalls durch Halogen substituiertes Phenyl und Phenoxy; sowie für gegebenenfalls substituiertes Phenylalkyl mit 1 bis 4 Kohlenstoffatomen im Alkylteil, wie insbesondere Benzyl und 1-Phenyl-eth-1-yl, wobei

als Phenylsubstituenten vorzugsweise infrage kommen: Halogen, Alkyl mit 1 bis 4 Kohlenstoffatomen, sowie Halogenalkyl mit 1 bis 2 Kohlenstoffatomen und 1 bis 5 gleichen oder verschiedenen Halogenatomen, wie insbesondere Fluor- und Chloratomen und wobei als Alkylsubstituenten vorzugsweise infrage kommen: Cyano, Hydroxycarbonyl und Alkoxycarbonyl mit 1 bis 4 Kohlenstoffatomen im Alkylteil.

Besonders bevorzugt sind diejenigen Verbindungen der Formel (I), in denen R für geradkettiges oder verzweigtes Alkyl mit 1 bis 8 Kohlenstoffatomen; für gegebenenfalls durch Methyl und Ethyl substituiertes Cyclohexyl, Cyclohexenyl, Cyclopentenyl, Cyclopentyl, Cyclohexyl-methyl, Cyclohexyl-ethyl, 2-Cyclohexyl-prop-1-yl, Cyclohexenyl-methyl, Cyclohexenyl-ethyl oder 2-Cyclohexenyl-prop-1-yl steht; für Methacryl, Trimethylvinyl, Acetylenyl, Hydroxyacetylenyl, Methoxyacetylenyl, Phenacetylenyl, Chlorphenylacetylenyl, Propargyl, Hydroxypropargyl, Methoxypropargyl, Phenylpropargyl oder Chlorphenylpropargyl steht; für gegebenenfalls durch Fluor, Chlor, Methyl, Phenyl, Phenoxy, Chlorphenyl und Chlorphenoxy substituiertes Phenyl steht; sowie für gegebenenfalls im Phenylteil durch Fluor, Chlor und Methyl substituiertes Benzyl oder 1-Phenyl-eth-1-yl steht, wobei die Methyl- bzw. Ethyl-Gruppe außerdem substituiert sein kann durch Cyano, Hydroxy- oder Methoxycarbonyl.

0048828

Im einzelnen seien außer den bei den Herstellungsbeispielen genannten Verbindungen die folgenden Verbindungen der allgemeinen Formel (I) genannt:

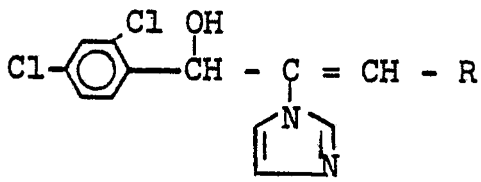 (I)

mit R = $-CH_3$

$-C_2H_5$

$-C_3H_7$-n

$-C_7H_{15}$-n

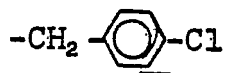

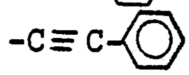

$-C(CH_3)_3$

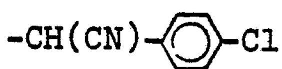

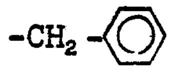

$-CH_2-C(CH_3)=CH_2$

$-C_4H_9$-n

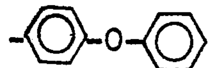

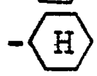

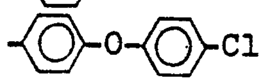

Verwendet man beispielsweise 3-Cyclohexyl-1-(2,4-dichlorphenyl)-2-(imidazol-1-yl)-2-propen-1-on und Natriumborhydrid als Ausgangsstoffe, so kann der Reaktionsablauf durch das folgende Formelschema wiedergegeben werden:

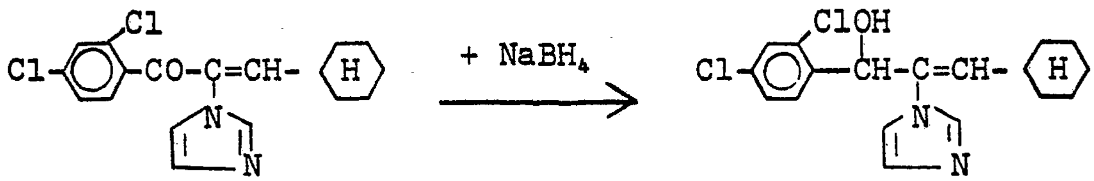


Die als Ausgangsstoffe für das erfindungsgemäße Verfahren zu verwendenden 2,4-Dichlorphenyl-imidazolyl-vinyl-ketone sind durch die Formel (II) allgemein definiert. In dieser Formel steht R vorzugsweise für diejenigen Reste, die bereits im Zusammenhang mit der Beschreibung der erfindungsgemäßen Stoffe der Formel (I) vorzugsweise für diesen Substituenten genannt wurden.

Die 2,4-Dichlorphenyl-imidazolyl-vinyl-ketone der Formel (II) sind noch nicht bekannt. Sie können jedoch gemäß einem eigenen Vorschlag oder in bekannter Art und Weise erhalten werden, indem man

a) Ketoenamine der Formel

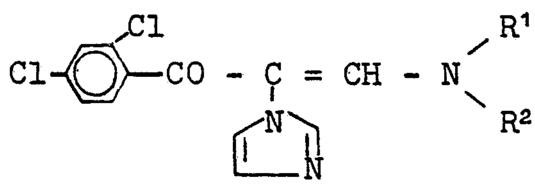

(III)

in welcher

$R^1$ und $R^2$ gleich oder verschieden sind und für Alkyl mit 1 bis 4 Kohlenstoffatomen, insbesondere Methyl, stehen,

mit magnesium-organischen Verbindungen der Formel

Hal - Mg - R (IV)

in welcher

R die oben angegebene Bedeutung hat und

Hal für Halogen, insbesondere Chlor oder Brom stehen,

in Gegenwart eines inerten organischen Lösungsmittels, wie beispielsweise Ether, und gegebenenfalls in Gegenwart eines Inertgases, wie beispielsweise Stickstoff, bei Temperaturen zwischen -20 und +120°C in üblicher Weise umsetzt, oder

b) 2,4-Dichlorphenacyl-imidazol der Formel

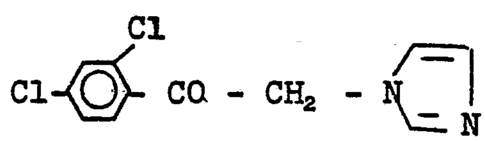

(V)

mit Aldehyden der Formel

O = CH - R (VI)

in welcher

R die oben angegebene Bedeutung hat,

in Gegenwart eines inerten organischen Lösungsmittels, wie beispielsweise Toluol, und in Gegenwart eines Katalysators, wie beispielsweise Piperidin/Essigsäure oder Benzoesäure, bei Temperaturen zwischen 20 und 160°C umsetzt.

Die Ketoenamine der Formel (III) sind noch nicht bekannt;sie können erhalten werden, indem man 2,4-Dichlorphenacyl -imidazol der Formel (V) mit Amidacetalen bzw. Aminalestern der Formeln

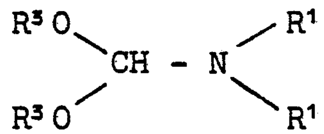 (VIIa)

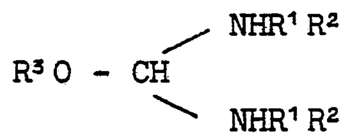 (VIIb)

in welchen

$R^1$ und $R^2$ die oben angegebene Bedatung haben und

$R^3$ für Alkyl mit 1 bis 4 Kohlenstoffatomen steht,

in an sich bekannter Art und Weise in Gegenwart eines inerten organischen Lösungsmittels, wie beispielsweise aromatische Kohlenwasserstoffe und wie insbesondere ein Ueberschuß an eingesetzten Amidacetalen bzw. Aminalester der Formeln (VIa) bzw. (VIb), in der Siedehitze umsetzt (vergleiche hierzu auch Chem.Ber. 101, 41-50 (1968); J.Org.Chem. 43, 4248-50 (1978) sowie die Herstellungsbeispiele).

Die Amidacetale und Aminalester der Formeln (VIIa) bzw. (VIIb) sind allgemein bekannte Verbindungen der organischen Chemie (vergleiche z.B. Chem.Ber.101, 41-50

(1968) und J.Org.Chem. 43, 4248-50 (1978)).

Die magnesiumorganischen Verbindungen der Formel (IV), des 2,4-Dichlorphenacyl-imidazol der Formel (V) und die Aldehyde der Formel (VI) sind bekannt.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei -10 bis + 30°C, vorzugsweise bei -10 bis 20°C durchgeführt. Hierzu setzt man auf 1 Mol des Ketons der Formel(II) etwa 1 Mol eines komplexen Hydrids, wie Natriumborhydrid, Calciumborhydrid oder Lithiumalanat, ein.

Zur Isolierung der erfindungsgemäßen Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert

werden; im allgemeinen arbeitet man zwischen 20 und 120°C, vorzugsweise bei 50 bis 100°C. Zur Durchführung der Reaktion setzt man auf 1 Mol des entsprechenden Ketones der Formel (II) etwa 1 bis 2 Mol Aluminiumisopropylat ein. Zur Isolierung der erfindungsgemäßen Verbindungen der Formel (I) wird das überschüssige Lösungsmittel durch Destillation im Vakuum entfernt und die entstandenen Aluminium-Verbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxyearbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren,wie z.B. p-Toluolsulfonsäure und 1,5-Naphthalindisulfonsäure.
Die Säureadditionssalze der Verbindungen der Formel(I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung bekämpfbaren Keime dar, sondern hat nur erläuternden Charakter.

Als Indikatonsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:
Alle Dermatomykosen und Systemmykosen, insbesondere solche, die durch die obengenannten Erreger hervorgerufen werden.

0048828

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees,Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten , Dragees, Kapseln, Pillen, Granulate, Suppositorien, Lösungen, Suspensionen und Emulsionen,

Pasten, Salben, Gele, Cremes, Lotions, Puder und Sprays genannt.

Tabletten ., Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchtehaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler oder Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesamöl, Glycerin, Glycerinformal, Tetrahydrofurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süßmittel, z.B. Saccharin enthalten.

Die therapeutisch wirksamen Verbindungen sollten in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen nach weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

0048828

Herstellungsbeispiele

Beispiel 1

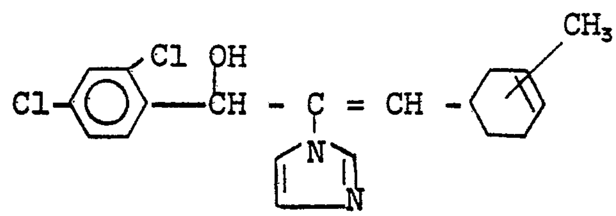

Zu 18,1g (0,05 Mol) 1-(2,4-Dichlorphenyl)-2-(imidazol-1-yl)-3-(methylcyclohexen-4-yl)-2-propen-1-on in 100ml Methanol gibt man 3,05g (0,025 Mol) Calciumchlorid und kühlt das Gemisch auf -20°C ab. Dazu werden portionsweise 1,9g (0,05 Mol) Natriumborhydrid gegeben und nach beendeter Zugabe auf -10°C erwärmt. Danach läßt man ein Aceton-Wasser-Gemisch zutropfen und läßt auf Raumtemperatur erwärmen. Das Reaktionsgemisch wird mit verdünnter Salzsäure auf einen pH-Wert von 5 eingestellt und eingeengt. Der Rückstand wird in einem Gemisch von Methylenchlorid/Wasser aufgenommen, die organische Phase abgetrennt, über Natriumsulfat getrocknet, filtriert und eingeengt. Man erhält 15,6g (85,7 % der Theorie) 1-(2,4-Dichlorphenyl)-2-(imidazol-1-yl)-3-(methylcyclohexen-4-yl)-2-propen-1-ol als amorphes Pulver.

Herstellung des Ausgangsproduktes

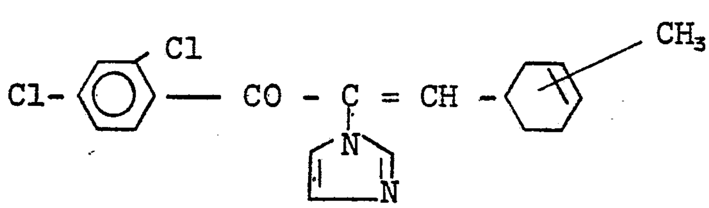

19,1 g (0,075 Mol) 2,4-Dichlorphenacyl-imidazol, 9,3 g (0,075 Mol) Methylcyclohexen-aldehyd, 1,83 g ( 20 Mol %) Benzoesäure und 0,96 g (15 Mol %) Piperidin werden in einem Gemisch aus 75 ml Cyclohexan und 50 ml Toluol aufgenommen und eine Stunde am Wasserabscheider unter Rückfluß erhitzt. Nach dem Abkühlen wird das Reaktionsgemisch mit verdünnter Natriumhydrogencarbonatlösung und zweimal mit Wasser gewaschen, die organische Phase wird abgetrennt, über Natriumsulfat getrocknet, filtriert und eingeengt. Das zurückbleibende Öl wird entgast. Man erhält 27 g ( 99 % der Theorie) 1-(2,4-Dichlorphenyl)-2-(imidazol-1-yl)-3-(methylcyclohexen-4-yl)-2-propen-1-on als zähes Öl.

Analog können die folgenden Verbindungen der allgemeinen Formel (I)

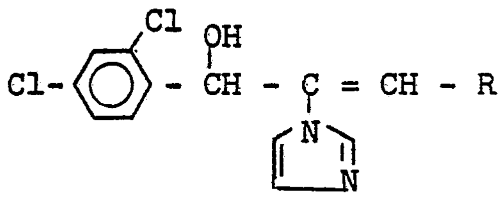 (I)

erhalten werden:

| Bsp.Nr. | R | physikalische Konstante |
|---|---|---|
| 2 | 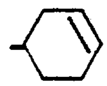 | amorph |
| 3 | $-CH(C_2H_5)_2$ | amorph |
| 4 | $-CH(CH_3)-C_3H_7-n$ | amorph |
| 5 | $-CH_2-CH(CH_3)-$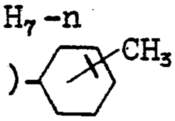 | amorph |
| 6 | $-CH(C_2H_5)-C_4H_9-n$ | amorph |
| 7 | $-C_6H_{13}-n$ | amorph |
| 8 | $-CH(CH_3)-$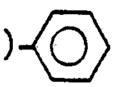 | amorph |
| 9 | $-CH(CH_3)-C_2H_5$ | amorph |
| 10 | 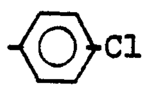 | amorph |
| 11 | $-C_2H_5$ | zähes Öl |

Anwendungsbeispiele

In dem nachfolgenden Beispiel wird die nachstehend angegebene aus der DE-OS 28 38 847 bekannte Verbindung als Vergleichssubstanz eingesetzt:

(A) = 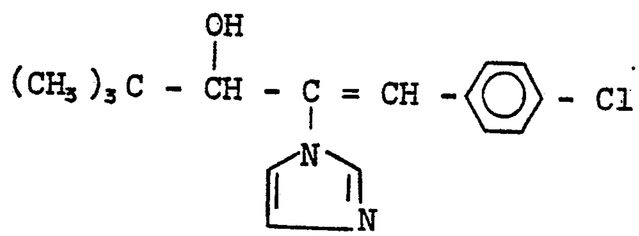

## Beispiel A

## Antimykotische in-vitro-Wirksamkeit

**Versuchsbeschreibung:**

Die in-vitro-Prüfungen wurden im Reihenverdünnungstest mit Keiminokula von durchschnittlich $5 \times 10^4$ Keimen/ml Substrat durchgeführt. Als Nährmedium dienten

a) für Dermatophyten und Schimmelpilze: Sabouraud's milieu d'épreuve

b) für Hefen: Fleischextrakt-Traubenzucker-Bouillon

Die Bebrütungstemperatur betrug 20°C, die Bebrütungsdauer lag bei 24 bis 96 Stunden.

In diesem Test zeigen insbesondere die Beispiele 1, 2, 3, 4, 5, 6, 9 und 10 eine bessere antimykotische Wirkung als die aus dem Stand der Technik bekannte Verbindung (A).

Patentansprüche

1. Substituierte 2,4-Dichlorphenyl-imidazolyl-vinyl-carbinole der allgemeinen Formel I

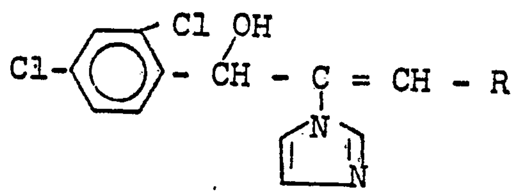

(I)

in welcher

R für Alkyl, gegebenenfalls substituiertes Cycloalkyl und Cycloalkenyl, gegebenenfalls substituiertes Cycloalkylalkyl und Cycloalkenylalkyl, gegebenenfalls substituiertes Alkenyl und Alkinyl, gegebenenfalls substituiertes Phenyl, sowie gegebenenfalls im Alkylteil und im Phenylteil substituiertes Phenylalkyl steht,

und deren physiologisch verträglichen Säureadditionssalze.

2. Substituierte 2,4-Dichlorphenyl-imidazolyl-vinyl-carbinole der allgemeinen Formel I in Anspruch 1, in welcher

R vorzugsweise für geradkettiges oder verzweigtes Alkyl mit 1 bis 29, insbesondere bis 18, Kohlenstoffatomen; für gegebenenfalls durch Alkyl mit 1 bis 4 Kohlenstoffatomen substituiertes Cycloalkyl und Cycloalkenyl mit jeweils 5 bis 7 Kohlenstoffatomen; für gegebenenfalls durch Alkyl mit 1 bis 4 Kohlenstoffatomen substituiertes Cycloalkylalkyl und Cycloalkenylalkyl mit jeweils 5 bis 7 Kohlenstoffatomen im Cycloalkyl- bzw. Cycloalkenyl-Teil und jeweils 1 bis 6 Kohlenstoffatomen im geradkettigen oder verzweigten Alkyl-Teil; für gegebenenfalls substituiertes geradkettiges oder verzweigtes Alkenyl und Alkinyl mit jeweils bis zu 6 Kohlenstoffatomen, wobei als Substituenten genannt seien: Hydroxy, Alkoxy mit 1 bis 4 Kohlenstoffatomen, sowie Phenyl, das gegebenenfalls substituiert sein kann durch Halogen und Alkyl mit 1 bis 4 Kohlenstoffatomen; für gegebenenfalls substituiertes Phenyl, wobei als Substituenten infrage kommen: Halogen, Alkyl mit 1 bis 4 Kohlenstoffatomen, Halogenalkyl mit 1 bis 2 Kohlenstoffatomen und 1 bis 5 gleichen oder verschiedenen Halogenatomen, wie insbesondere Fluor- und Chloratomen, sowie gegebenenfalls durch Halogen substituiertes Phenyl und Phenoxy; sowie für gegebenenfalls substituiertes Phenylalkyl mit 1 bis 4 Kohlenstoffatomen im Alkylteil, wie insbesondere Benzyl und 1-Phenyl-eth-1-yl, wobei als Phenylsubstituenten vorzugsweise infrage kommen: Halogen, Alkyl mit 1 bis 4 Kohlenstoffatomen, sowie Halogenalkyl mit 1 bis 2 Kohlenstoffatomen und 1 bis 5 gleichen oder verschiedenen Halogenatomen, wie insbesondere Fluor- und Chloratomen und wobei als Alkylsubstituenten vorzugsweise infrage kommen: Cyano, Hydroxycarbonyl und Alkoxycarbonyl mit 1 bis 4 Kohlenstoffatomen im Alkylteil.

3. Substituierte 2,4-Dichlorphenyl-imidazolyl-vinyl-carbinole der allgemeinen Formel I in Anspruch 1,

in welcher

R für geradkettiges oder verzweigtes Alkyl mit 1 bis 8 Kohlenstoffatomen; für gegebenenfalls durch Methyl und Ethyl substituiertes Cyclohexyl, Cyclohexenyl, Cyclopentenyl, Cyclopentyl, Cyclohexyl-methyl, Cyclohexyl-ethyl, 2-Cyclohexyl-prop-1-yl, Cyclohexenyl-methyl, Cyclohexenyl-ethyl oder 2-Cyclohexenyl-prop-1-yl steht; für Methacryl, Trimethylvinyl, Acetylenyl, Hydroxyacetylenyl, Methoxyacetylenyl, Phenacetylenyl, Chlorphenylacetylenyl, Propargyl, Hydroxypropargyl, Methoxypropargyl, Phenylpropargyl oder Chlorphenylpropargyl steht; für gegebenenfalls durch Fluor, Chlor, Methyl, Phenyl, Phenoxy, Chlorphenyl und Chlorphenoxy substituiertes Phenyl steht; sowie für gegebenenfalls im Phenylteil durch Fluor, Chlor und Methyl substituiertes Benzyl oder 1-Phenyl-eth-1-yl steht, wobei die Methyl- bzw. Ethyl-Gruppe außerdem substituiert sein kann durch Cyano, Hydroxy- oder Methoxycarbonyl.

4. 1-(2,4-Dichlorphenyl)-2-(imidazol-1-yl)-3-(methyl-cyclohex-3-enyl)-2-propen-1-ol.

5. 1-(2,4-Dichlorphenyl)-2-imidazol-1-yl)-3-(cyclohex-3-enyl)-2-propan-1-ol.

6. 1-(2,4-Dichlorphenyl)-2-(imidazol-1-yl)-4-ethyl-2-hexen-1-ol.

7. Verfahren zur Herstellung von substituierten 2,4-Dichlorphenyl-imidazolyl-vinyl-carbinolen der Formel (I) in Anspruch 1, dadurch gekennzeichnet, daß man 2,4-Dichlorphenyl-imidazolyl-vinyl-ketone der Formel

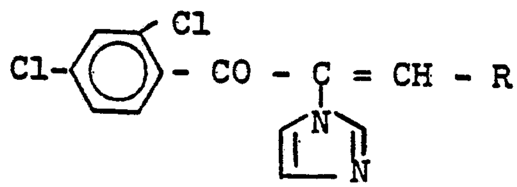

(II)

in welcher

R die oben angegebene Bedeutung hat,

in an sich bekannter Weise reduziert.

8. Arzneimittel, gekennzeichnet durch einen Gehalt an mindestens einem substituierten 2,4-Dichlorphenyl-imidazolyl-vinyl-carbinol gemäß Anspruch 1.

9. Verfahren zur Herstellung von antimykotischen Mitteln, dadurch gekennzeichnet, daß man substituierte 2,4-Dichlorphenyl-imidazolyl-vinyl-carbinole gemäß Anspruch 1 mit inerten, nichttoxischen, pharmazeutisch geeigneten Trägerstoffen vermischt.

10. Verfahren zur Behandlung von Mykosen, dadurch gekennzeichnet, daß man substituierte 2,4-Dichlorphenyl-imidazolyl-vinyl-carbinole gemäß Anspruch 1 Menschen oder Tieren appliziert, die an Mykosen erkrankt sind.

Europäisches Patentamt

# EUROPÄISCHER RECHERCHENBERICHT

0048828

Nummer der Anmeldung
EP 81 10 6723

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | CHEMICAL ABSTRACTS, Band 94, Nr. 17, 27. April 1981, Seite 767, Nr. 139814c<br>Columbus, Ohio, U.S.A.<br>& JP - A - 80 111 477 (A.G. BAYER) 28-08-1980<br>* Zusammenfassung | 1-10 |
| | & EP - A - 0 032 239<br>-- | |
| | GB - A - 1 504 352 (I.C.I.)<br>* Insgesamt * | 1-10 |
| | --<br>US - A - 4 182 862 (HAK-FOON CHAN)<br>* Insgesamt * | 1-10 |
| | ---- | |

KLASSIFIKATION DER ANMELDUNG (Int. Cl.³)

C 07 D 233/60
A 61 K 31/415

RECHERCHIERTE SACHGEBIETE (Int. Cl.³)

C 07 D 233/60

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-01-1982 | ALLARD |